(19) Europäisches Patentamt
European Patent Office
Office européen des brevets

(11) **EP 2 536 437 B1**

(12) **EUROPÄISCHE PATENTSCHRIFT**

(45) Veröffentlichungstag und Bekanntmachung des Hinweises auf die Patenterteilung:
**13.05.2020 Patentblatt 2020/20**

(21) Anmeldenummer: **11704970.0**

(22) Anmeldetag: **17.02.2011**

(51) Int Cl.:
*A61K 49/04* (2006.01)          *A61K 6/00* (2020.01)

(86) Internationale Anmeldenummer:
**PCT/EP2011/052366**

(87) Internationale Veröffentlichungsnummer:
**WO 2011/101418 (25.08.2011 Gazette 2011/34)**

(54) **INFILTRATIONSLÖSUNG ZUR BEHANDLUNG EINER ZAHNSCHMELZLÄSION**

INFILTRATION SOLUTION FOR TREATING AN ENAMEL LESION

SOLUTION D'INFILTRATION POUR LE TRAITEMENT D'UNE LÉSION DE L'ÉMAIL DENTAIRE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **17.02.2010 DE 202010003032 U**

(43) Veröffentlichungstag der Anmeldung:
**26.12.2012 Patentblatt 2012/52**

(73) Patentinhaber: **Mühlbauer Technology GmbH**
**22547 Hamburg (DE)**

(72) Erfinder:
• **NEANDER, Sven**
**20148 Hamburg (DE)**
• **KÖNIG, Thomas**
**20255 Hamburg (DE)**
• **NEFFGEN, Dr. Stephan**
**25421 Pinneberg (DE)**
• **PARIS, Sebastian**
**24251 Osdorf (DE)**
• **MEYER-LÜCKEL, Hendrik**
**24631 Langwedel (DE)**

(74) Vertreter: **Glawe, Delfs, Moll**
**Partnerschaft mbB von**
**Patent- und Rechtsanwälten**
**Postfach 13 03 91**
**20103 Hamburg (DE)**

(56) Entgegenhaltungen:
**EP-A1- 2 108 356          EP-A1- 2 153 812**
**EP-A2- 2 151 229          WO-A1-90/03036**
**US-A1- 2006 247 329       US-A1- 2007 142 495**
**US-A1- 2008 182 921**

• **PATTEN H.E.:**
**"https://pubs.acs.org/doi/pdf/10.1021/j150 061a002", ACS.org , 22 June 1904 (1904-06-22), Retrieved from the Internet: URL:https://pubs.acs.org/doi/pdf/10.1021/j 150061a002 [retrieved on 2019-01-31]**
• **A.N. Kirgintsev: "Solubility of tetraphenylborates of the alkali metals and ammonium in acetone-water mixtures at 25°", Bulletin of the Academy of Sciences of the USSR, Division of chemical science , 1 June 1968 (1968-06-01), Retrieved from the Internet: URL:https://link.springer.com/article/10.1 007/BF01106247 [retrieved on 2019-01-31]**
• **WYNN D A ET AL: "The solubility of alkali-metal fluorides in non-aqueous solvents with and without crown ethers, as determined by flame emission spectrometry.", TALANTA NOV 1984, vol. 31, no. 11, November 1984 (1984-11), pages 1036-1040, ISSN: 0039-9140**
• **Richard Buchner: "Dielectric Spectroscopy of Cesium Fluoride in Methanol", Journal of Solution Chemistry; July 2002, Volume 31, Issue 7, pp 521-535 , July 2002 (2002-07), Retrieved from the Internet: URL:https://link.springer.com/article/10.1 023/A:1020217511766 [retrieved on 2019-01-31]**

**Beschreibung**

Die Erfindung betrifft eine Infiltrationslösung einer röntgenopaken Metallverbindung, einen Kit für die Dentalapplikation sowie dessen Verwendung zur Prävention bzw. Behandlung (Versiegelung) kariöser Schmelzläsionen. Der Kit enthält als erste Komponente die erfindungsgemäße Infiltrationslösung einer röntgenopaken Metallverbindung und als zweite Komponente einen auszuhärtenden Infiltranten, enthaltend polymerisations- bzw. vernetzungsfähige Monomere.

Unter kariösen Schmelzläsionen sind hier im Wesentlichen sich im Zahnschmelz ausdehnende kariöse Schädigungen zu verstehen, die noch nicht zu Kavitation (Lochbildung) geführt haben. Kariöse Schmelzläsionen sind demineralisierte Bereiche des Zahnschmelzes, die eine Tiefe bis zu 2-3 mm haben können. Das Porenvolumen eines Läsionskörpers kann dabei 5 bis 25 % betragen.

Aus WO 2007/131725 A1 sind zur Behandlung kariöser Schmelzläsionen ein Infiltrationsverfahren und Infiltranten bekannt, so dass die Kavitation verhindert wird und sich die sonst üblicherweise erfolgende Restauration mit Dentalkompositen erübrigt. Bei dem Infiltrationsverfahren wird, nachdem eine eventuell vorhandene remineralisierte oberflächliche Schicht entfernt wurde, die Läsion mit einem Infiltranten, der im Wesentlichen aus Monomeren besteht, in Kontakt gebracht, woraufhin diese infiltrieren. Nachdem der Infiltrant die Läsion durchdrungen hat, werden die Monomere mittels Lichtaktivierung polymerisiert. Die Läsion ist dann versiegelt. Das Fortschreiten der Karies wird gestoppt.

Für die Infiltration sind spezielle Monomere bzw. Monomermischungen erforderlich, da bekannte Dentalkleber für Dentalkomposite (auch Adhäsive oder Bondings genannt) zu langsam und/oder nicht ausreichend in die Läsion eindringen und/oder die Läsion vollständig durchdringen (penetrieren oder infiltrieren). WO 2007/131725 A1 beschreibt die Verwendung Monomerer bzw. von Monomermischungen, so dass der Infiltrant einen Penetrationskoeffizienten PK > 50 cm/s hat.

Nachteilig an den aus dem Stand der Technik (z.B. WO 2007/131725 A1) bekannten Infiltranten ist deren unzureichende Röntgenopazität. Sie sind für Röntgenstrahlen im Wesentlichen durchlässig (transluzent) und daher bei einer Röntgendiagnostik nicht oder nur sehr schwer erkennbar. Damit ist eines der wichtigsten Instrumente zur Erkennung des Ausmaßes und der Lage vorhandener Infiltrationen entwertet. Abgesehen davon ist es bei Einsatz der Röntgendiagnostik schwierig, auf Grund der unzureichenden Röntgenopazität der Infiltranten eine evtl. unter der infiltrierten Läsion weiter fortschreitende Karies festzustellen, da sie nicht oder sehr schlecht vom infiltrierten Bereich unterschieden werden kann. Um eine fortschreitende Karies festzustellen, sind dann aufwändige genau reproduzierbare Bissflügelaufnahmen erforderlich, wie sie in der deutschen Gebrauchsmusteranmeldung DE 202008006814 U1 beschrieben sind.

EP 2 153 812 A1 (nicht vorveröffentlicht) offenbart, in einen Infiltranten, der vernetzende Monomere enthält, zusätzlich röntgenopake Materialien einzubringen.

EP 2 151 229 B1 betrifft einen Infiltranten für die Dental-applikation, der vernetzende Monomere enthält sowie dessen Verwendung zur Behandlung bzw. Prävention von kariösen Schmelzläsionen.

Der Erfindung liegt die Aufgabe zu Grunde, eine Möglichkeit zu schaffen, die Röntgenopazität von infiltrierten bzw. zu infiltrierenden Bereichen eines Zahns weiter zu verbessern.

Gelöst wird diese Aufgabe durch einen Kit gemäß Anspruch 1 aus einer erfindungsgemäßen Infiltrationslösung und einem Infiltranten, der polymerisierbare bzw. vernetzende Monomere enthält. Vorteilhafte Weiterbildungen der Erfindung sind in den Unteransprüchen angegeben.

Die Erfindung hat erkannt, dass durch Infiltration restaurierte Zahnbereiche mittels Röntgendiagnostik nicht leicht zu identifizieren sind und möglicherweise durch eine höhere Röntgenopazität der infiltrierten Läsion ein für die röntgendiagnostische Untersuchung ausreichender Kontrast zum umgebenden Zahn- und Knochengewebe hergestellt werden könnte.

Die unzureichende Kontrastierung, wie sie für Infiltrationen festgestellt wurde, stellt sich bei den herkömmlich verwendeten Dentalmaterialien, wie sie bei der Restauration oder dem Zahnersatz zu Einsatz kommen, nicht. Die im Stand der Technik verwendeten metallischen Restaurationen erzeugen bereits von sich aus einen guten Kontrast. Gleiches gilt für keramische Materialien und Kunststoffkomposite, bei denen die vorrangig aus Gründen der Erhöhung der mechanischen Festigkeit und der Reduzierung des Schrumpfs zugesetzten Füllstoffe und/oder Pigmente einen ausreichenden röntgenopaken Kontrast bereitstellen.

Kern der vorliegenden Erfindung ist es, eine Infiltrationslösung zur Verfügung zu stellen, mit deren Hilfe sich vor der Durchführung der eigentlichen Infiltration mit vernetzenden Monomeren bereits im Vorfeld röntgenopake Materialien in die Läsion einbringen lassen, die den Röntgenkontrast des infiltrierten Bereichs erhöhen. Die erfindungsgemäße Infiltrationslösung dringt in eine Läsion mindestens etwa in gleichem Maße ein wie ein Infiltrant, der vernetzende Monomere enthält. Das flüchtige Lösungsmittel verdampft/verdunstet, so dass die darin gelösten röntgenopaken Stoffe in der Läsion zurückbleiben und deren Röntgenkontrast erhöhen. Bei Bedarf kann die erfindungsgemäße Infiltrationslösung mehrfach hintereinander angewendet werden, um mehr röntgenopake Materialien in die behandelte Läsion einzubringen und so deren Röntgenkontrast auf das gewünschte Maß zu erhöhen.

Die erfindungsgemäß eingesetzte röntgenopake Metallverbindung ist in dem Lösungsmittel oder Lösungsmittelgemisch gelöst. Es kann sich erfindungsgemäß um eine echte Lösung oder aber eine kolloidale Lösung handeln.

Zur Herstellung einer echten Lösung können beispielsweise metallorganische Verbindungen oder Metallsalze verwendet werden, die in dem entsprechenden Lösungsmittel im gewünschten Umfang löslich sind.

Zur Herstellung einer kolloidalen Lösung können geeignete nanoskalige Füllstoffe verwendet werden. Der Begriff kolloidale Lösung wird hier so verwendet wie definiert in Ullmann's Encyclopedia of Industrial Chemistry, 6. Auflage, Band 9, S. 31ff. Bei kolloidalen Lösungen können somit als Kolloid in der Lösung dispergierte nanoskalige Füllstoffe mit geeignetem Röntgenkontrast vorliegen. Üblicherweise spricht man von einer kolloidalen Lösung, wenn die Teilchengrößen der kolloidalen Partikel zwischen 1 nm und 1 $\mu$m liegen. Bei der erfindungsgemäß verwendeten kolloidalen Lösung kann es sich somit insbesondere um eine kolloidale Dispersion eines nanoskaligen Füllstoffs in einem geeigneten (flüchtigen) Lösungsmittel handeln. Bevorzugt im Zusammenhang mit der vorliegenden Erfindung sind kolloidale Dispersionen von Partikeln mit Teilchengrößen von 1 bis 100 nm.Besonders bevorzugt sind nicht aggregierte oder agglomerierte nanoskalige Füllstoffe.

Die vorliegende Erfindung hat erkannt, dass sich Infiltrationslösungen mit röntgenopaken nanoskaligen Füllstoffen und/oder röntgenopaken sonstigen anorganischen oder organischen Metallverbindungen, insbesondere salzartigen Metallverbindungen zur bestimmungsgemäßen Verwendung bei Dentalapplikationen bereitstellen lassen, wenn diese als echte oder kolloidale Lösung vorliegen.

Weiterhin hat die Erfindung erkannt, dass sich der röntgenopake Kontrast weiter verbessern lässt, wenn die Infiltrationslösungen nanoskaliger Füllstoffe und/oder sonstiger anorganischer oder organischer Metallverbindungen, insbesondere salzartiger Metallverbindungen, separat von dem zu härtenden Infiltranten (gegebenenfalls in mehreren vorgelagerten Schritten) in die Läsion eingebracht werden.

In einem ersten Schritt wird eine Infiltrationslösung enthaltend ein flüchtiges Lösungsmittel und die gelöste röntgenopake Verbindung und in einem zweiten Schritt ein im Wesentlichen Monomere und/oder im Wesentlichen Monomere und eine gelöste röntgenopake Metallverbindung enthaltender zu härtender Infiltrant angewendet.

Zunächst seien einige im Rahmen der Erfindung verwendete Begriffe erläutert.

Der Begriff Infiltrant bezeichnet eine Flüssigkeit, die in eine Zahnschmelzläsion (einen porösen Festkörper) eindringen kann. Ein Infiltrant enthält oder besteht aus polymerisierbaren und/oder vernetzbaren Monomeren. Nach dem Eindringen in eine Läsion kann der Infiltrant dort unter Polymerisation und/oder Vernetzung der Monomere ausgehärtet werden.

Von einem solchen Infiltranten zu unterscheiden ist eine erfindungsgemäße Infiltrationslösung, deren wesentlichen Bestandteile lediglich flüchtiges Lösungsmittel und die in Anspruch 1 definierten Metallverbindungen sind. Die Applikation einer solchen erfindungsgemäßen Infiltrationslösung dient somit ausschließlich dazu, vor der Anwendung des eigentlichen Infiltranten röntgenopake Materialien in die Läsion zu transportieren. Das als Vehikel für den Transport der röntgenopaken Materialien in die Läsion verwendete Lösungsmittel wird anschließend verdunstet oder verdunsten gelassen.

Das Eindringen einer Flüssigkeit (z.B. ungehärtetes Harz (Infiltrant) oder Infiltrationslösung, nachfolgend auch allgemein als flüssiges Harz bezeichnet) in einen porösen Festkörper (Zahnschmelzläsion) wird durch die Washburn-Gleichung (Gleichung 1, siehe unten) physikalisch beschrieben. Bei dieser Gleichung wird angenommen, dass der poröse Festkörper ein Bündel offener Kapillaren darstellt (Buckton G., Interfacial phenomena in drug delivery and targeting. Chur, 1995); in diesem Fall wird das Eindringen der Flüssigkeit von Kapillarkräften getrieben.

$$d^2 \approx \left( \frac{\gamma \cdot \cos\theta}{2\eta} \right) r \cdot t \qquad - \text{Gleichung 1} -$$

d     Abstand, um den sich das flüssige Harz bewegt
$\gamma$     Oberflächenspannung des flüssigen Harzes (gegen Luft)
$\theta$     Kontaktwinkel des flüssigen Harzes (gegen Zahnschmelz)
$\eta$     dynamische Viskosität des flüssigen Harzes
r     Kapillarradius (Porenradius)
t     Eindringdauer

Der in Klammern stehende Ausdruck der Washburn-Gleichung wird als Penetrationskoeffizient (PK, Gleichung 2, siehe unten) bezeichnet (Fan P. L. et al., Penetrativity of sealants. J. Dent. Res., 1975, 54: 262-264). Der PK setzt sich aus der Oberflächenspannung der Flüssigkeit gegen Luft (y), dem Cosinus des Kontaktwinkels der Flüssigkeit gegen Zahnschmelz ($\theta$) und der dynamischen Viskosität der Flüssigkeit ($\eta$) zusammen. Je größer der Wert des Koeffizienten ist, um so schneller dringt die Flüssigkeit in eine gegebene Kapillare oder ein gegebenes poröses Bett ein. Dies bedeutet, dass ein hoher Wert des PK durch hohe Oberflächenspannungen, niedrige Viskositäten und niedrige Kontaktwinkel erzielt werden kann, wobei der Einfluss des Kontaktwinkels vergleichsweise gering ist.

$$PK = \left( \frac{\gamma . \cos \theta}{2\eta} \right) \qquad - \text{Gleichung 2} -$$

PK     Penetrationskoeffizient
$\gamma$     Oberflächenspannung des flüssigen Harzes (gegen Luft)
$\theta$     Kontaktwinkel des flüssigen Harzes (gegen Zahnschmelz)
$\eta$     dynamische Viskosität des flüssigen Harzes

Infiltrationslösungen vorliegender Erfindung enthalten in Lösungsmittel gelöste röntgenopake Metallverbindungen (echte oder kolloidale Lösung).

Gegenstand der Erfindung ist somit ein Kit umfassend eine Infiltrationslösung zur Behandlung einer Zahnschmelzläsion, die enthält:

(a) wenigstens 25 Gew.-% eines bei Raumtemperatur (23°C) flüchtigen Lösungsmittels oder Lösungsmittelgemisches, und
(b) eine in dem Lösungsmittel oder Lösungsmittelgemisch gelöste röntgenopake Metallverbindung, die eine Röntgenopazität größer 200 % Aluminium bestimmt gemäß EN ISO 4049:2000 aufweist.

Ein erfindungsgemäßes Kit zur Behandlung einer Zahnschmelzläsion umfasst eine erfindungsgemäße Infiltrationslösung und einen härtbaren Infiltranten. Gegenstand der Erfindung ist somit auch ein Kit aus einer erfindungsgemäßen Infiltrationslösung und einem Infiltranten des Standes der Technik. Geeignete härtbare Infiltranten sind beispielsweise bekannt aus WO 2007/131725 A1, deren Offenbarung durch Bezugnahme darauf auch zum Gegenstand der vorliegenden Anmeldung gemacht wird.

Die Erfindung wird weiterhin verwirklicht durch ein Verfahren zur Infiltration einer Zahnschmelzläsion, umfassend die Schritte:

(1) Einlagerung einer röntgenopaken Metallverbindung in die Läsion mittels einer erfindungsgemäßen Infiltrationslösung, deren Lösungsmittel anschließend verdunstet oder verdunsten gelassen wird,
(2) Infiltration der so vorbehandelten Läsion mit einem Infiltranten, der polymerisierbare und/oder vernetzbare Monomere enthält,
(3) Erhärten des Infiltranten in der Läsion.

Die Infiltrationslösung zur Verwendung für Schritt 1 hat einen hohen Penetrationskoeffizienten PK und durchdringt die Läsion in kurzer Zeit vollständig, das Lösungsmittel wird verdunsten gelassen, die röntgenopake Metallverbindung bleibt zurück und setzt sich in der Läsion ab. Der Schritt (1) kann bei Bedarf mehrfach wiederholt werden, um eine hinreichende Menge röntgenopaken Metallverbindungen in die Läsion einzubringen.

Der Infiltrant zur Verwendung für Schritt 2 kann zusätzlich gelöste röntgenopake Verbindungen enthalten, wie beispielsweise offenbart in EP 2 153 812 A1. Als Lösungsmittel dienen dann das flüssige Harz bzw. die vernetzenden Monomere.

In einer Variante der Erfindung weisen die Infiltrationslösung des Schrittes (1) und der Infiltrant des Schrittes (2) gleiche oder ähnliche Penetrationskoeffizienten auf. Dies bewirkt, dass die Eindringtiefe der Infiltrationslösung in die Läsion in Schritt (1) gleich oder ähnlich ist wie die Eindringtiefe des Infiltranten in Schritt (2) bei der nachfolgenden eigentlichen Infiltration der Läsion mit vernetzbaren Monomeren. Auf diese Weise wird sichergestellt, dass die in Schritt (1) gewissermaßen eingebrachte Markierung mit röntgenopaken Stoffen ähnlich tief reicht wie die in Schritt (2) durchgeführte eigentliche Infiltration mit Monomeren, die anschließend ausgehärtet werden. Gemäß dieser Variante weisen somit auch bei einem erfindungsgemäßen Kit die Infiltrationslösung und der Infiltrant gleiche oder ähnliche Penetrationskoeffizienten auf.

Der PK-Wert der Infiltrationslösung liegt bevorzugt über 50 cm/s, weitere bevorzugte Untergrenzen sind 100, 200 und 300 cm/s. Die erreichbare Obergrenze kann bspw. bei 1.000 oder 900 cm/s liegen, u.a. abhängig vom verwendeten Lösungsmittel. Der Infiltrant der erfindungsgemäßen Kits kann ebenfalls die genannten Mindest-PK-Werte aufweisen, wird jedoch u.U. niedrigere PK-Werte als die Infiltrationslösung aufweisen, so dass erreichbare Obergrenzen von bspw. 600, 500, 400 oder 300 cm/s vorliegen können.

Durch die erfindungsgemäße Infiltrationslösung und das erfindungsgemäße Verfahren kann der Gehalt röntgenopaker Verbindungen in infiltrierten Läsionen um ein Vielfaches erhöht werden, wodurch infiltrierte Bereiche besser mittels Röntgendiagnostik sichtbar gemacht werden können. Bevorzugt ist die Röntgenopazität des infiltrierten Läsionskörpers deutlich größer als die des (gesunden) Zahnschmelzes. Es ist jedoch auch möglich, die Röntgenopazität der infiltrierten Läsion an die des Zahnschmelzes anzupassen, so dass nur nicht infiltrierte Läsionsbereiche bzw. eine weiter fortschreitende Karies röntgendiagnostisch zu erkennen wäre. Insbesondere soll die Schaffung eines röntgenopakeren infiltrierten Läsionsbereich verhindern, dass eine mit einem Infiltranten behandelte Läsion in einer nachträglichen röntgenographischen Untersuchung fälschlicherweise als aktive kariöse Läsion diagnostiziert wird, was bei Behandlung mit einer Füllungstherapie zu unnötigem Substanzverlust am Zahn führen würde. Jede deutliche Erhöhung der Röntgenopazität der Läsion, die den Mineralverlust der Läsion gegenüber dem unverletzten Schmelz teilweise oder vollständig kompensiert oder sogar überkompensiert, ist damit wünschenswert. Bevorzugt ist die Röntgenopazität der Läsion nach Behandlung mit dem erfindungsgemäßen Kit daher > 100% Al, besonders bevorzugt mindestens im Bereich des gesunden Zahnschmelzes und ganz besonders bevorzugt größer als die das gesunden Zahnschmelzes.

Die Menge und/oder Auswahl der einzubringenden röntgenopaken Verbindungen ist bei dem erfindungsgemäßen Verfahren weniger beschränkt, als bei Einbringen einer röntgenopaken Verbindung nur zusammen mit dem zu härtenden, flüssige Harze enthaltenden Infiltranten.

Geeignete röntgenopake Metallverbindungen sind im Lösungsmittel der Infiltrationslösung löslich.

Bei den erfindungsgemäß geeigneten nanoskaligen Füllstoffen handelt es sich um Metallverbindungen, insbesondere um Metall- oder Mischmetalloxide, -silikate, -nitride, -sulfate, -titanate, -zirkonate, -stannate, -wolframate oder um eine Mischung aus diesen Verbindungen. Unter dem Begriff Mischmetalloxid, -nitrid, usw. ist hier eine chemische Verbindung zu verstehen, in der mindestens zwei Metalle und/oder Halbmetalle zusammen mit dem entsprechenden (Nicht-)metallanion (Oxid, Nitrid, usw.) chemisch miteinander verbunden sind.

Bei den erfindungsgemäß einsetzbaren nanoskaligen Füllstoffen handelt es sich bevorzugt um Zirkondioxid, Zinkoxid, Zinndioxid, Ceroxid, Silizium-Zink-Oxide, Silizium-Zirkon-Oxide, Indiumoxide und deren Mischungen mit Siliziumdioxid und/oder Zinndioxid, Strontiumsulfat, Bariumsulfat, Strontiumtitanat, Bariumtitanat, Natriumzirkonat, Kaliumzirkonat, Magnesiumzirkonat, Calciumzirkonat, Strontiumzirkonat, Bariumzirkonat, Natriumwolframat, Kaliumwolframat, Magnesiumwolframat, Calciumwolframat, Strontiumwolframat und/oder Bariumwolframat.

Besonders bevorzugt eingesetzte nanoskalige röntgenopake Füllstoffe sind ausgewählt aus der Gruppe bestehend aus Salzen der Seltenerdmetalle, des Scandiums, des Yttriums, des Bariums und Strontiums, oder Wolframaten. Als schwerlösliche Salze eignen sich vorzugsweise Sulfate, Phosphate oder Fluoride.

Unter den Salzen der Seltenerdmetalle (Elemente 57-71), des Scandiums oder des Yttriums sind die Trifluoride bevorzugt. Zu den bevorzugten Seltenerdmetallen zählen Lanthan, Cer, Samarium, Gadolinium, Dysprosium, Erbium oder Ytterbium. Unter deren Salzen sind die Fluoride bevorzugt, insbesondere Ytterbiumtrifluorid ($YbF_3$). Bevorzugte Barium- und Strontiumsalze sind Fluoride, Phosphate und Sulfate, insbesondere die Sulfate.

Der Ausdruck "Wolframat" umfasst Metallverbindungen der Orthowolframate und Polywolframate, wobei erstere bevorzugt sind.

Das Metallwolframat ist vorzugsweise eine Wolframatverbindung eines mehrwertigen Metalls, insbesondere eines zwei- oder dreiwertigen Metalls. Geeignete zweiwertige Metalle umfassen Erdalkalimetalle, wie Magnesium, Calcium, Strontium oder Barium, insbesondere Calcium, Strontium oder Barium. Strontium- und Bariumwolframate zeichnen sich durch eine besonders hohe Röntgenopazität aus, da in diesen Verbindungen zwei gute Kontrastbildner miteinander kombiniert werden. Bevorzugte dreiwertige Metalle umfassen Scandium, Yttrium oder Seltenerdmetalle, wie Lanthan, Cer, Samarium, Gadolinium, Dysprosium, Erbium oder Ytterbium. Auch hier ergibt sich eine besonders hohe Röntgenopazität dadurch, dass ein guter Kontrastbildner (Wolframat) mit einem stark kontrastbildenden Metall kombiniert wird. Darüber hinaus ist es möglich, die erfindungsgemäß eingesetzten Wolframate (bzw. auch die anderen nanoskaligen Salze) mit Metallatomen zu dotieren. Vorzugsweise wird hierzu das Wirtsgittermetall durch den Dotierungsstoff in einer Menge von bis zu 50 Mol-%, stärker bevorzugt 0,1 bis 40 Mol-%, noch stärker bevorzugt 0, 5 bis 30 Mol-%, insbesondere 1 bis 25 Mol-% ersetzt. Der gewählte Dotierungsstoff kann zur Röntgenopazität beitragen. Aus analytischen Gründen kann es jedoch auch von Interesse sein, eine oder mehrere dotierende Metalle zu wählen, die lumineszierende Eigenschaften, insbesondere Photolumineszenz vermitteln. Zu diesem Zweck geeignete Dotierungsstoffe sind fachbekannt und werden oft unter einem vom Wirtsgittermetall verschiedenen Lanthanid ausgewählt. Beispiele umfassen die kombinierte Dotierung mit Eu und Bi, oder die Dotierung von Ce in Kombination mit Nd, Dy oder Tb, oder Er in Kombination mit Yb. Gleichermaßen kann man ein Wolframat als Wirtsgitter mit einem geeigneten Lanthanidion oder einem anderen Metallion, z. B. $Bi^{3+}$ oder $Ag^+$ dotieren.

Die erfindungsgemäßen nanoskaligen röntgenopaken Füllstoffe weisen vorzugsweise mittlere Partikelgrößen $d_{50}$ bzw. Bereiche dieser Partikelgrößen von kleiner 100 nm, bevorzugt kleiner 25 nm, oder zwischen 1 nm und 80 nm, zwischen 4 nm und 60 nm, zwischen 6 nm und 50 nm, zwischen 0,5 nm und 22 nm, zwischen 1 nm und 20 nm, zwischen 1 nm und 10 nm oder zwischen 1 nm und 5 nm auf.

Besonders bevorzugt sind vereinzelt vorliegende, nicht aggregierte und nicht agglomerierte nanoskalige Füllstoffe.

Weiterhin bevorzugt sind Füllstoffe mit unimodaler Teilchengrößenverteilung. Die Begriffe Aggregat und Agglomerat werden so verwendet wie in DIN 53206 definiert.

Der erfindungsgemäße nanoskalige Füllstoff weist eine BET-Oberfläche (gemäss DIN 66131 bzw. DIN ISO 9277) zwischen 15 m$^2$/g und 600 m$^2$/g, bevorzugt zwischen 30 m$^2$/g und 500 m$^2$/g und besonders bevorzugt zwischen 50 m$^2$/g und 400 m$^2$/g auf.

Die nanoskaligen Füllstoffe liegen als kolloidale oder echte Lösungen vor.

Geeignete röntgenopake Metallverbindungen für echte Lösungen sind gut lösliche (ionische) anorganische Metallsalze, wie Halogenide und Nitrate etc. Bevorzugt sind Fluoride, Chloride, Iodide und Bromide. Bevorzugte Metalle sind die der röntgenopaken Füllstoffe. Besonders geeignet sind bspw. Cäsiumfluorid, Rubidiumfluorid, Zinkbromid etc.

Geeignete röntgenopake Metallverbindungen echter Lösungen sind weiterhin gut lösliche (ionische) organische Salze von Carbonsäureestern, z.B. Acetate oder Alkoholate, z.B. Ethanolate etc. Es kann bevorzugt sein, dass die organischen Salze polymerisierbar sind, wie Acrylate und Methacrylate (Monomere). Beispiele geeigneter Monomere sind Zirkoniumacrylat, Zirkonyldimethacrylat, Zirkoniumtetra-(meth)acrylat, Zirkoniumcaboxyethyl(meth)acrylat, Zirkonium (bromonorbornanlactoncarboxylat)tri(meth)acrylat, Hafnium(meth)acrylat, Hafniumcaboxyethyl(meth)acrylat, Strontium(meth)acrylat, Barium(meth)acrylat, Ytterbium(meth)acrylat oder Yttrium(meth)acrylat.

Geeignete röntgenopake Metallverbindungen sind weiterhin kovalente organische Metallverbindungen wie Triphenylbismut-verbindungen.

Die gelöste Metallverbindung liegt zu mehr als 5 %, bevorzugt zu mehr als 20 %, weiter bevorzugt zu mehr als 25 % besonders bevorzugt zu 30 bis 65 % in der Infiltrationslösung vor. Diese Prozentangaben sind Gewichtsprozent, sofern nicht anders definiert.

Die Metallverbindung hat eine Röntgenopazität gemäß Messvorschrift im Verfahren nach EN ISO 4049:2000 bevorzugt größer 300 % Aluminium, bevorzugt größer 500 % Aluminium, am bevorzugtesten größer 700 % Aluminium.

Erfindungsgemäß geeignete Lösungsmittel oder Lösungsmittelgemische sind solche, in denen die röntgenopaken Metallverbindungen gut löslich bzw. kolloidal löslich sind.

Hinsichtlich des Schritts 1 des erfindungsgemäßen Verfahrens geeignete Lösungsmittel oder Lösungsmittelgemische sind solche, die gut aus der Läsion verdunsten können.

Bevorzugte leicht verdunstende Lösungsmittel sind protische oder polare aprotische Lösungsmittel. Geeignete Lösungsmittel haben bei 20°C einen Dampfdruck von > 10 hPa, bevorzugt > ca. 20 hPa, weiter bevorzugt > 30 hPa. Ein besonders bevorzugter Bereich von Dampfdrücken des Lösemittels bzw. der Zubereitung zur Infiltration liegt zwischen 30 bis 300 hPa, insbesondere 30 bis 100 hPa. Besonders geeignete Lösungsmittel haben eine geringe Molekülmasse (< 120 g/mol). Die Lösungsmittel sind bevorzugt ausgewählt aus der Gruppe der Alkohole (bevorzugt Alkanole), Ketone, Ether oder Ester. Geeignete Lösemittel sind bspw. Methanol, Ethanol, 2-Propanol, 1-Propanol, Butanol, 2-Methyl-2-propanol, Dimethylether, Ethylmethylether, Diethylether, Tetrahydrofuran, Propanon, Butanon, Ethylacetat und Propylacetat. Die Lösungsmittel können allein oder als Mischungen verwendet werden.

Die Lösungsmittel haben bevorzugt eine Verdunstungszahl kleiner 35 nach DIN 53170:2009. Besonders bevorzugte Lösungsmittel haben eine Verdunstungszahl kleiner 20, noch bevorzugter kleiner 10.

Das zu verdunstende Lösungsmittel kann gleichzeitig als Trockenmittel für die Läsion fungieren. Besonders bevorzugtes Lösungsmittel ist Ethanol.

Die Infiltrationslösung kann dentalübliche oder sonstige Additive wie Initiatoren, Beschleuniger, Stabilisatoren, Inhibitoren, Filmbildner, Farbstoffe, Fluoreszenzfarbstoffe, Antibiotika, Fluoridierungsmittel, Remineralisierungsmittel, Tenside sowie Chelatkomplexbildner und/oder Kristallisationshemmer enthalten.

Die Infiltrationslösung kann Bestandteil eines Kits zur Behandlung einer Zahnschmelzläsion sein. Das Kit umfasst mindestens die erfindungsgemäße Infiltrationslösung und einen härtbaren Infiltranten.

Als härtbare Infiltranten sind die des Standes der Technik geeignet.

Das Kit kann ein Ätzmittel umfassen. Geeignet sind die Ätzmittel des Standes der Technik.

Das Kit kann ein zusätzliches Trockenmittel enthalten.

Dem erfindungsgemäßen Verfahren kann ein Ätzschritt vorausgehen, um eine oberflächliche Schicht der Läsion zu entfernen. Das Ätzmittel wird entfernt und die Läsion getrocknet.

Die Infiltrationslösung wird aufgetragen und infiltriert die Läsion, sodass das Porenvolumen vollkommen ausgefüllt ist. Das Lösungsmittel wird verdunsten gelassen. Die Verdunstung kann durch Maßnahmen wie Luftstrom, Wärmezufuhr etc. unterstützt werden.

Die Schritte des Infiltrationsverfahrens, insbesondere der Schritt (1), können mehrmals wiederholt werden, um den Gehalt röntgenopaker Verbindungen in der Läsion weiter zu erhöhen.

Nach einer ersten und/oder zweiten Infiltration kann optional ein Füllstoffe enthaltender Versiegler oder Lack aufgetragen werden, der bevorzugt einen Penetrationskoeffizienten PK unter 50 cm/s aufweist, mit dem Infiltranten kompatibel ist, mit diesem zusammen oder separat ausgehärtet wird und einen guten Verbund herstellt. Der Versiegler oder Lack enthält bevorzugt die erfindungsgemäßen röntgenopaken nanoskaligen Füllstoffe, bevorzugt in höheren Gehalten als der Infiltrant. Der Versiegler oder Lack kann jedoch auch andere Füllstoffe aufweisen bspw. barium- oder strontiumhaltige inerte Dentalgläser und/oder Ionomergläser.

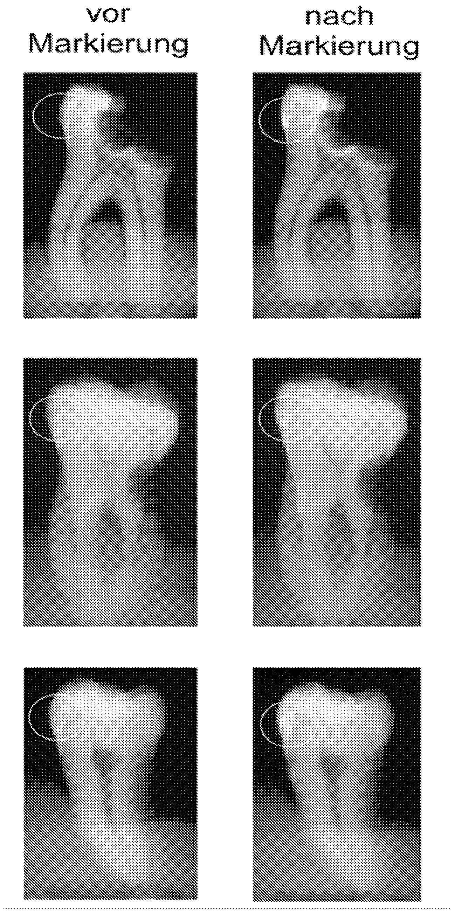

Im Folgenden ist die Erfindung anhand einiger Beispiele erläutert. Figur 1 zeigt Röntgenaufnahmen natürlicher Läsionen von Molaren vor und nach einer erfindungsgemäßen Behandlung.

Verwendete Substanzkürzel und ihre Bedeutung:

| TEDMA | Triethlenglycoldimethacrylat |
|---|---|
| UDMA | Urethandimethacrylat (*CAS* 72869-86-4) |
| Bis-GMA | Bis-Phenol-A-Glycidyldimethacrylat (*CAS* 1565-94-2) |
| E3TMPTA | Ethoxyliertes Trimetylolpropantriacrylat |
| CQ | Kampherchinon |
| EHA | Ethylhexyl-p-N,N-dimethylaminobenzoat |
| BHT | 2,6-Di-tert.-butylphenol |
| LTPO | Lucerin® TPO (BASF) |

Prüfungsmethoden

Oberflächenspannung

Die Oberflächenspannung der Infiltranten wurde mittels Konturanalyse eines hängenden Tropfens durchgeführt (DSA 10, KRÜSS GmbH). Die Messung der Oberflächenspannung wurde an neu gebildeten Tropfen über einen Zeitraum von 30 s durchgeführt, wobei etwa alle 5 s ein Messwert aufgenommen wurde. Hierzu wurden die Harze mit einer feinen Spritze ausgebracht und der sich bildende Tropfen mit einer Digitalkamera gefilmt. Aus der charakteristischen Form und Größe des Tropfens wurde die Oberflächenspannung gemäß der Young-Laplace-Gleichung bestimmt. Für jedes Harz wurden so 3 Messungen durchgeführt, deren Mittelwert als Oberflächenspannung angegeben wurde.

Dichtebestimmung

Die Dichten der Infiltranten wurden mittels Pyknometer bestimmt. Hierzu wurde die Dichte der Luft mit 0,0013 g/ml und die Erdbeschleunigung mit 9,8100 m/s$^2$ berücksichtigt.

Kontaktwinkel

Für jede Einzelmessung wurde Schmelz aus Rinderzähnen verwendet. Hierzu wurden Rinderzähne in einem Kunstharz eingebettet und die Schmelzfläche mittels Schleifmaschine (Struers GmbH) mit Schleifpapieren (80, 500 und 1200 Körnung) nass poliert, so dass plane etwa 0,5x1,0 cm große Schmelzflächen für die Kontaktwinkel-Messungen zur Verfügung standen. Die Schmelzproben wurden bis zur Messung in destilliertem Wasser gelagert und vor der Messung mit Ethanol und Druckluft getrocknet.

Die Messung des Kontaktwinkels erfolgte mit Hilfe eines Video-Kontaktwinkelmessgerät (DSA, KRÜSS GmbH). Hierbei wurde mittels Mikroliterspritze ein Tropfen des Infiltranten auf die Schmelzfläche gebracht, innerhalb von 10 s bis zu 40 Einzelaufnahmen des Tropfens rechnergesteuert aufgenommen und per Tropfenkonturanalyse-Software der Kontaktwinkel ermittelt.

Dynamische Viskosität

Die Viskosität der Harze wurde bei 23°C mittels dynamischen Platte-Platte-Viskosimeter (Dynamic Stress Rheometer, Rheometric Scientific Inc.) gemessen. Es wurde im "Steady Stress Sweep"-Modus bei Spaltgrößen von 0,1 bis 0,5 mm im Bereich von 0 bis 50 Pa Schubspannung ohne Vorscherung der Harze gemessen.

Röntgenopazität

Die Messung der Röntgenopazität erfolgte durch Röntgenbestrahlung von ca. 1 mm dicken Probekörpern gemäß den Vorgaben der EN ISO 4049:2000 (Füllungs-, restaurative und Befestigungskunststoffe). Für die Bestimmung der Röntgenopazität von Materialien aus denen sich für sich genommen durch Aushärtung keine Probekörper herstellen ließen, wurden aushärtbare Komposite hergestellt. Hiervon ließen sich Die Röntgenopazität der röntgenopaken Verbindung (100 %) wurde aus einer Auftragung des Gewichtsanteils röntgenopaker Verbindung im Prüfkörper/Komposit gegen die gemessene Röntgenopazität graphisch durch Extrapolation auf 100% röntgenopaker Verbindung ermittelt.

Beispiel 1

Zunächst wurde die Röntgenopazität von Triphenylbismut (Ph3Bi) sowie folgender Salze ermittelt: Cäsiumfluorid (CsF), Cäsiumiodid (CsI), Bariumfluorid (BaF2), Strontiumfluorid (SrF2), Ytterbiumfluorid (YbF3), Yttriumfluorid (YF3), Strontiumchlorid (SrC12), Zinkbromid (ZnBr2) und Rubidiumfluorid (RbF).

Die genannten Salze wurden jeweils zu 20, 40 und 60 Gewichtsprozent in lichthärtendem Harz (40 Gew-% Bis-GMA, 20 Gew-% UDMA, 20 Gew-% TEDMA, 20 Gew-% ethoxyliertes Bis-GMA, 0,2 Gew-% CQ, 0,2 Gew-% EHA, 0,2 Gew-% LTPO und 0,005 Gew% BHT) zusammen mit 2% Aerosil® (R812S) homogenisiert. Hierzu wurden alle Komponenten per SpeedMixer (Fa. Hauschild) zweimal bei 3000 U/min vermischt. Anschließend wurden die Pasten auf einem Dreiwalzenstuhl dispergiert und noch einmal für 20s bei 3000 U/min per SpeedMixer vermischt. Eventuell vorhandene Luftblasen wurden durch ein kurzzeitiges Entgasen der Paste im Exsikkator entfernt.

Direkt nach Herstellung der Pasten/Komposite wurden gemäß EN ISO 4049:2000 1 mm dicke Probekörper durch Belichtung hergestellt. Die exakte Probenkörperdicke wurde mittels Schieblehre festgestellt. Die Probekörper wurden zusammen mit einem Aluminiumstufenkeil (Reinheitsgrad >98% Aluminium, mit weniger als 0,1% Kupfer- und weniger als 1% Eisenanteil) auf einen Röntgenfilm (Zahnfilm Ultraspeed DF-50, Filmempfindlichkeit D, Fa. Kodak) gestellt. Probekörper, Aluminiumstufenkeil und Film wurden mit einem analogen einphasigen Röntgengerät der Firma Gendex im Abstand von 400 mm für 0,4 s mit Röntgenstrahlen einer Beschleunigungsspannung von 65 kV bestrahlt. Nach dem Entwickeln und Fixieren des Films wurden die Schwärzungsgrade der Abbildung der Probekörper und des Aluminiumstufenkeiles gemessen, eine Schwärzungskurve (Schwärzungsgrad gegen Aluminiumstufenhöhe) für den Aluminiumstufenkeil erstellt und die Werte der Röntgenopazitäten für jeden Probekörper graphisch ermittelt.

Weiterhin wurde die Röntgenopazität des lichthärtenden Komposits ohne (0 Gew-%) röntgenopakes Salz auf die gleiche Weise gemessen. Das Komposit bestand nur aus dem lichthärtenden Harz und 2% Aerosil (R812S).

Die Röntgenopazitätsprobekörper von Hafnium-carboxyethylacrylat wurden durch Aushärtung einer 60%-igen alkoholischen Lösunge hergestellt. Die Lösung enthielt zusätzlich Lichtstarter (1 Gew-Teil CQ, 1,6 Gew-Teile EHA) der per Magnetrührer bei Raumtemperatur innerhalb von einer Stunde gelöst wurde. Nach Einfüllen in die Form ließ man das Lösemittel verdunsten, um einen vermessbaren Prüfkörper zu erhalten.

Es ergaben sich folgende Röntgenopazitäten [in % Aluminium] für die hergestellten Komposite:

| Gew-% | YbF$_3$ | CsI | RbF | SrF$_2$ | ZnCl$_2$ |
|---|---|---|---|---|---|
| **0** | 19 | 19 | 19 | 19 | 19 |
| **20** | 99 | 87 | 130 | 110 | 128 |
| **40** | 287 | 266 | 329 | 260 | - |
| **60** | 530 | 489 | 484 | 468 | 450 |
| **Extrapolation auf 100 Gew-%** | >700 | >700 | >700 | >700 | >700 |

| Gew-% | BaF$_2$ | Ph$_3$Bi | CsF | SrCl$_2$ | YF$_3$ |
|---|---|---|---|---|---|
| **0** | 19 | 19 | 19 | 19 | 19 |
| **20** | 88 | 145 | 85 | 98 | 85 |
| **40** | 228 | 252 | 210 | 228 | 167 |
| **60** | 449 | 446 | 434 | 422 | 316 |
| **Extrapolation auf 100 Gew-%** | >700 | >700 | >700 | >700 | >500 |

| Gew-% | Hafnium-carboxyethyl-acrylat |
|---|---|
| **100** | 257 |

Zusätzlich wurde die Röntgenopazität von gesundem humanem Zahnschmelz nach der oben beschriebenen Röntgenopazitäts-Bestimmungsmethode gemessen. Sie betrug 158% Aluminium. Dieser Wert stimmt gut mit den Angaben der einschlägigen Literatur für Humanschmelz von etwa 160% Al überein.

Beispiel 2

Es wurde eine 40 Gew.-% CsF enthaltende ethanolische Lösung (Infiltrationslösung) sowie eine 15 Gew.-% CsF enthaltende polymerisierbare Lösung (härtbarer Infiltrant; 15% CsF, 67,5% TEDMA, 16,9% E3TMPTA, 0,2% EHA, 0,2% CQ, 0,2% LTPO und 0,005% BHT) hergestellt. Nach kurzer Rührzeit lagen klare Lösungen vor.

Der Penetrationskoeffizient der ethanolischen 40%igen CsF-Infiltrationslösung betrug ca. 900 cm/s. Die Verdunstungszahl nach DIN 53170:2009 war 8.

Der Penetrationskoeffizient des härtbaren 15%igen CsF-Infiltranten betrug ca. 150 cm/s.

Beispiel 3

Verdunstungsversuche

Zur weiteren Beurteilung der Eignung von Lösungsmittel aus Schmelzläsionen möglichst schnell und vollständig zu verdunsten, wurde in einem ersten Versuch der Verdunstungsgrad [%] bei 37°C (Mundtemperatur) bestimmt. Hierbei wurden jeweils 0,1g des Lösungsmittels auf einem Kristallisierschälchen (d=40mm) bei Raumtemperatur verdunsten gelassen. Der Gewichtsverlust wurde in Abhängigkeit von der Zeit [s] auf einer Analysenwaage verfolgt und ins Verhältnis zur Ausgangsmenge gebracht. Die Menge des eingesetzten Lösungsmittels entspricht in etwa der Menge, die für eine Infiltrationsbehandlungen zu verwenden ist.

In einem zweiten Versuch (bei 23°C) wurde das Lösungsmittel zusätzlich im Luftstrom mit 2 bar Druck verblasen. Die Verdunstungsgrade [%] durch Verblasen bei der höheren Mundtemperatur müssten dann noch höher sein, als die hier dargestellten Werte.

Tabelle: Verdunstungsgrade:

|          |          | 60 s   | 120 s  | 180 s  |
|----------|----------|--------|--------|--------|
| Ethanol  | 37°C     |        | > 90 % | 100 %  |
|          | Luftstrom| > 80 % |        |        |
| Propanol | 37°C     |        | 80     |        |
|          | Luftstrom| > 90 % |        |        |

Beispiel 4

In dem nachfolgenden Beispiel wurden natürliche approximale Läsionen dreier Molarer (Humanzähne)behandelt.

Als Infiltrationslösung wurde eine Lösung von 21 Gew.% CsF in Ethanol verwendet.

Ein polymerisierbarer Infiltrant wurde hergestellt gemäß EP 2145613 A1, Tabelle Harz 2, in dem 0,5 Gew.-% CQ, 0,84 Gew.-% EHA und 0,002 Gew.-% BHT durch Rühren unter Gelblichtbedingungen gelöst werden. Nach Herstellung wurde der Infiltrant bis zur Verwendung unter Lichtausschluss gelagert.

Durchführung des erfindungsgemäßen Verfahrens:

Die approximale Läsion wurde 30 s mit der Infiltrationslösung in Kontakt gebracht. Anschließend wurde die Läsion 30 s einem Strom ölfreier Luft ausgesetzt. Die Behandlung wurde dreimal wiederholt.

Als nächstes wurde zweimal hintereinander eine Infiltration mit polymerisierbarem Infiltranten durchgeführt. Die Anwendungsdauer des Infiltranten bei der ersten Infiltration betrug 3 min, bei der zweiten Infiltration 1 min. Nach jedem Infiltrationsschritt wurde die infiltrierte Läsion je 40 s Blaulicht einer LED-Polymerisationslampe ausgesetzt. Dies führte zur sicheren Aushärtung des Infiltranten.

Von den Molaren wurden vor und nach Durchführung des erfindungsgemäßen Verfahrens standardisierte Röntgenaufnahmen hergestellt (konventioneller F-Film, 70 kV, 0,4 s). Die Aufnahmen sind in Figur 1 gezeigt.

Bei allen drei Molaren ist vor der erfindungsgemäßen Behandlung jeweils eine approximale kariöse Läsion als röntgenologische Aufhellung sichtbar (in Figur 1 mit einem Kreis markiert). Nach der erfindungsgemäßen Behandlung ist eine deutliche Zunahme der Radioopazität von allen drei Läsionen zu erkennen (Fig.1). Hierbei lag die Röntgendichte der behandelten Läsionen über der des umgebenden Schmelzes.

**Patentansprüche**

1. Kit zur Behandlung einer Zahnschmelzläsion, umfassend [1] einen härtbaren Infiltranten, enthaltend polymerisations- bzw. vernetzungsfähige Monomere, und [2] eine Infiltrationslösung zur Behandlung einer Zahnschmelzläsion, **dadurch gekennzeichnet, dass** die Infiltrationslösung enthält:

   (a) wenigstens 25 Gew.-% eines bei Raumtemperatur (23°C) flüchtigen Lösungsmittels oder Lösungsmittelgemisches, und
   (b) eine in dem Lösungsmittel oder Lösungsmittelgemisch gelöste röntgenopake Metallverbindung, die eine Röntgenopazität größer 200 % Aluminium bestimmt nach EN ISO 4049:2000 aufweist,

   wobei die Infiltrationslösung zu mindestens 80 Gew.-% aus dem Lösungsmittel oder Lösungsmittelgemisch (a) und der gelösten Metallverbindung (b) besteht.

2. Kit nach Anspruch 1, **dadurch gekennzeichnet, dass** die Infiltrationslösung bei Raumtemperatur (23°C) einen Penetrationskoeffizienten PK größer 100 cm/s, bevorzugt größer 200 cm/s, besonders bevorzugt größer 300 cm/s aufweist.

3. Kit nach Anspruch 1 oder 2, **dadurch gekennzeichnet, dass** das Lösungsmittel oder Lösungsmittelgemisch (a) eine Verdunstungszahl VD kleiner 35, bevorzugt kleiner 10 nach DIN 53170:2009 aufweist.

4. Kit nach einem der Ansprüche 1 bis 3, **dadurch gekennzeichnet, dass** die Infiltrationslösung zu mindestens 90 Gew.-%, bevorzugt zu mindestens 95 Gew.-% aus dem Lösungsmittel oder Lösungsmittelgemisch (a) und der gelösten Metallverbindung (b) besteht.

5. Kit nach einem der Ansprüche 1 bis 4, **dadurch gekennzeichnet, dass** das Lösungsmittel oder Lösungsmittelgemisch (a) ausgewählt ist aus der Gruppe bestehend aus Alkoholen, Ethern, Ketonen und Estern.

6. Kit nach einem der Ansprüche 1 bis 5, **dadurch gekennzeichnet, dass** die Infiltrationslösung mindestens 30 Gew.-%, vorzugsweise 35 bis 70 Gew.-% des Lösungsmittels oder Lösungsmittelgemischs (a) enthält.

7. Kit nach einem der Ansprüche 1 bis 6, **dadurch gekennzeichnet, dass** die gelöste Metallverbindung (b) eine Röntgenopazität größer 300 % Aluminium, vorzugsweise größer 500 % Aluminium, weiter vorzugsweise größer 700 % Aluminium aufweist.

8. Kit nach einem der Ansprüche 1 bis 7, **dadurch gekennzeichnet, dass** die gelöste Metallverbindung (b) die Verbindung eines Metalls einer Atomnummer von 30 oder höher, bevorzugt mit einer Ordnungszahl von 38 bis 83 ist.

9. Kit nach einem der Ansprüche 1 bis 8, **dadurch gekennzeichnet, dass** die gelöste Metallverbindung (b) ein Salz ist.

10. Kit nach einem der Ansprüche 1 bis 8, **dadurch gekennzeichnet, dass** die gelöste Metallverbindung eine metallorganische Verbindung ist.

11. Kit nach einem der Ansprüche 1 bis 10, **dadurch gekennzeichnet, dass** die Infiltrationslösung mindestens 5 Gew.-%, vorzugsweise mindestens 20 Gew.-%, weiter vorzugsweise mindestens 25 Gew.-%, weiter vorzugsweise 30 bis 65 Gew.-% der gelösten Metallverbindung enthält.

12. Kit nach einem der Ansprüche 1 bis 11, **dadurch gekennzeichnet, dass** die Infiltrationslösung die gelöste Metallverbindung in kolloidaler Lösung enthält.

13. Kit nach einem der Ansprüche 1 bis 11, **dadurch gekennzeichnet, dass** die Infiltrationslösung die gelöste Metallverbindung in echter Lösung enthält.

14. Kit nach einem der Ansprüche 1 bis 13 zur Behandlung einer kariösen Schmelzläsion.

**Claims**

1.  Kit for treating an enamel lesion, comprising [1] a curable infiltrant, comprising polymerizable or crosslinkable monomers, and [2] an infiltration solution for treating an enamel lesion, **characterized in that** the infiltration solution comprises:

    (a) at least 25% by weight of a solvent or solvent mixture which is volatile at room temperature (23°C), and
    (b) dissolved in solution in the solvent or solvent mixture, a radiopaque metal compound having a radiopacity of more than 200% aluminum as determined in accordance with EN ISO 4049:2000,

    wherein the infiltration solution is composed to an extent of at least 80% by weight of the solvent or solvent mixture (a) and of the dissolved metal compound (b)

2.  Kit according to Claim 1, **characterized in that** the infiltration solution at room temperature (23°C) has a penetration coefficient PC of greater than 100 cm/s, preferably greater than 200 cm/s, more preferably greater than 300 cm/s.

3.  Kit according to Claim 1 or 2, **characterized in that** the solvent or solvent mixture (a) has an evaporation index EI of less than 35, preferably less than 10, according to DIN 53170:2009.

4.  Kit according to any of Claims 1 to 3, **characterized in that** the infiltration solution is composed to an extent at least 90% by weight, preferably at least 95% by weight of the solvent or solvent mixture (a) and of the dissolved metal compound (b).

5.  Kit according to any of Claims 1 to 4, **characterized in that** the solvent or solvent mixture (a) is selected from the group consisting of alcohols, ethers, ketones, and esters.

6.  Kit according to any of Claims 1 to 5, **characterized in that** the infiltration solution comprises at least 30% by weight, preferably 35% to 70% by weight, of the solvent or solvent mixture (a).

7.  Kit according to any of Claims 1 to 6, **characterized in that** the dissolved metal compound (b) has a radiopacity of greater than 300% aluminum, preferably greater than 500% aluminum, more preferably greater than 700% aluminum.

8.  Kit according to any of Claims 1 to 7, **characterized in that** the dissolved metal compound (b) is the compound of a metal with an atomic number of 30 or higher, preferably having an atomic number of 38 to 83.

9.  Kit according to any of Claims 1 to 8, **characterized in that** the dissolved metal compound (b) is a salt.

10. Kit according to any of Claims 1 to 8, **characterized in that** the dissolved metal compound is an organometallic compound.

11. Kit according to any of Claims 1 to 10, **characterized in that** the infiltration solution comprises at least 5% by weight, preferably at least 20% by weight, more preferably at least 25% by weight, more preferably 30 to 65% by weight of the dissolved metal compound.

12. Kit according to any of Claims 1 to 11, **characterized in that** the infiltration solution comprises the dissolved metal compound in colloidal solution.

13. Kit according to any of Claims 1 to 11, **characterized in that** the infiltration solution comprises the dissolved metal compound in true solution.

14. Kit according to any of Claims 1 to 13 for treating a carious enamel lesion.


**Revendications**

1.  Kit pour le traitement d'une lésion de l'émail dentaire, comprenant [1] un agent d'infiltration durcissable, contenant des monomères polymérisables ou réticulables et [2] une solution d'infiltration pour le traitement d'une lésion de l'émail dentaire, **caractérisé en ce que** la solution d'infiltration contient :

(a) au moins 25 % en poids d'un solvant ou mélange de solvants volatil à température ambiante (23 °C), et
(b) un composé métallique opaque aux rayons X dissous dans le solvant ou mélange de solvants, qui présente une opacité aux rayons X supérieure à 200 % d'aluminium déterminée selon EN ISO 4049:2000,

la solution d'infiltration étant constituée à hauteur d'au moins 80 % en poids par le solvant ou mélange de solvants (a) et le composé métallique dissous (b).

**2.** Kit selon la revendication 1, **caractérisé en ce que** la solution d'infiltration présente à température ambiante (23 °C) un coefficient de pénétration PK supérieur à 100 cm/s, de préférence supérieur à 200 cm/s, de manière particulièrement préférée supérieur à 300 cm/s.

**3.** Kit selon la revendication 1 ou 2, **caractérisé en ce que** le solvant ou mélange de solvants (a) présente un indice d'évaporation VD inférieur à 35, de préférence inférieur à 10, selon DIN 53170:2009.

**4.** Kit selon l'une quelconque des revendications 1 à 3, **caractérisé en ce que** la solution d'infiltration est constituée à hauteur d'au moins 90 % en poids, de préférence à hauteur d'au moins 95 % en poids, du solvant ou mélange de solvants (a) et du composé métallique dissous (b).

**5.** Kit selon l'une quelconque des revendications 1 à 4, **caractérisé en ce que** le solvant ou mélange de solvants (a) est choisi dans le groupe constitué par les alcools, les éthers, les cétones et les esters.

**6.** Kit selon l'une quelconque des revendications 1 à 5, **caractérisé en ce que** la solution d'infiltration contient au moins 30 % en poids, de préférence 35 à 70 % en poids du solvant ou mélange de solvants (a).

**7.** Kit selon l'une quelconque des revendications 1 à 6, **caractérisé en ce que** le composé métallique dissous (b) présente une opacité aux rayons X supérieure à 300 % d'aluminium, de préférence supérieure à 500 % d'aluminium, de manière davantage préférée supérieure à 700 % d'aluminium.

**8.** Kit selon l'une quelconque des revendications 1 à 7, **caractérisé en ce que** le composé métallique dissous (b) est le composé d'un métal d'un numéro atomique de 30 ou plus, de préférence ayant un numéro d'ordre de 38 à 83.

**9.** Kit selon l'une quelconque des revendications 1 à 8, **caractérisé en ce que** le composé métallique dissous (b) est un sel.

**10.** Kit selon l'une quelconque des revendications 1 à 8, **caractérisé en ce que** le composé métallique dissous est un composé métallo-organique.

**11.** Kit selon l'une quelconque des revendications 1 à 10, **caractérisé en ce que** la solution d'infiltration contient au moins 5 % en poids, de préférence au moins 20 % en poids, de manière davantage préférée au moins 25 % en poids, de manière davantage préférée 30 à 65 % en poids du composé métallique dissous.

**12.** Kit selon l'une quelconque des revendications 1 à 11, **caractérisé en ce que** la solution d'infiltration contient le composé métallique dissous en solution colloïdale.

**13.** Kit selon l'une quelconque des revendications 1 à 11, **caractérisé en ce que** la solution d'infiltration contient le composé métallique dissous en solution véritable.

**14.** Kit selon l'une quelconque des revendications 1 à 13 pour le traitement d'une lésion de l'émail cariée.

vor Markierung    nach Markierung

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2007131725 A1
- DE 202008006814 U1
- EP 2153812 A1
- EP 2151229 B1
- EP 2145613 A1

**In der Beschreibung aufgeführte Nicht-Patentliteratur**

- Ullmann's Encyclopedia of Industrial Chemistry. vol. 9, 31ff
- **BUCKTON G.** *Interfacial phenomena in drug delivery and targeting. Chur,* 1995
- **FAN P. L. et al.** Penetrativity of sealants. *J. Dent. Res.,* 1975, vol. 54, 262-264